Europäisches Patentamt

European Patent Office

Office européen des brevets

(11) Veröffentlichungsnummer: **0 296 484**
**A2**

(12) **EUROPÄISCHE PATENTANMELDUNG**

(21) Anmeldenummer: 88109609.3

(22) Anmeldetag: 16.06.88

(51) Int. Cl.⁴: **C12N 15/00 , C12N 1/20 ,**
**//(C12N1/20,C12R1:26)**

Patentansprüche für folgende Vertragsstaaten:
ES + GR.

(30) Priorität: 24.06.87 DE 3720834

(43) Veröffentlichungstag der Anmeldung:
28.12.88 Patentblatt 88/52

(84) Benannte Vertragsstaaten:
AT BE CH DE ES FR GB GR IT LI LU NL SE

(71) Anmelder: HOECHST AKTIENGESELLSCHAFT
Postfach 80 03 20
D-6230 Frankfurt am Main 80(DE)

(72) Erfinder: Winnacker, Ernst Ludwig, Prof. Dr.
Dall-Armistr. 41a
D- 8000 München 19(DE)
Erfinder: Metzler,Thomas
Augustenstr. 41
D-8000 München 2(DE)
Erfinder: Marquardt, Rüdiger, Dr.
Günthersburgallee 69
D 6000 Frankfurt(DE)
Erfinder: Präve, Paul, Prof. Dr.
Kronberger Weg 7
D 6231 Sulzbach(DE)

(54) Promotoren zur Expression von Fremd-DNA in methylotrophen Bakterien, deren Gewinnung und deren Verwendung.

(57) Aus nativen Plasmiden von Methylomonas clara können Promotorregionen zur Expression von Genen in E. coli und methylotrophen Mikroorganismen isoliert werden, die eine überraschend starke Transkriptionsaktivität besitzen.

EP 0 296 484 A2

# Promotoren zur Expression von Fremd-DNA in methylotrophen Bakterien, deren Gewinnung und deren Verwendung

Es ist bekannt, daß in methylotrophen Bakterien unter Verwendung verschiedener Vektorsysteme und unter Verwendung verschiedener Transcriptionssignale eine Expression von Fremd-DNA erreicht werden kann. Die hierfür verwendeten Transcriptionssignale stammen dabei entweder von Resistenzgenen oder aber sie entsprechen den in E. coli für eine Überexpression von Genen häufig verwendeten Promotoren, beispielsweise tac, lac, trp oder auch Phagen-Promotoren. Windass et al., Nature 287, 396 (1980) beschreiben die Klonierung und Expression von Glutamatdehydrogenase aus E. coli in Methylophilus methylotrophus; Hennam et al., Nature 297, 80 (1982) sowie DeMayer et al., Proc. Natl. Acad. Sci. 79, 4256 (1982) untersuchten die Expression von Ovalbumin und α1-Interferon in methylotrophen Mikroorganismen, um nur Beispiele zu nennen.

In der Europäischen Patentanmeldung 98967 wird die Entwicklung von Vektorsystemen für Methylomonas clara beschrieben. Native Plasmide des methylotrophen Mikroorganismus werden zur Herstellung von Hybridplasmiden verwendet, die sowohl in Methylomonas clara als auch in E. coli stabil replizieren. In der Deutschen Patentanmeldung 3635583 wird vorgeschlagen, derartige Hybridplasmide mit Hilfe von Fragmenten des nativen Methylomonas Plasmids herzustellen.

Die nativen Plasmide von Methylomonas clara sind cryptisch, d.h. man kann ihnen keine biologische Funktion zuweisen. Daher war es überraschend, daß Regionen mit starker Transkriptionsaktivität auf diesen Plasmiden lokalisiert werden konnten.

Die Erfindung betrifft somit:

1. Eine Promotorregion aus einem nativen Plasmid von Methylomonas clara, zur Expression von Genen in E. coli und methylotrophen Mikroorganismen.

2. Einen Hybridvektor, der die unter 1 definierte Promotorregion enthält.

3. Die Verwendung des nach 2 definierten Hybridvektors zur Herstellung von Polypeptiden in E. coli und in methylotrophen Mikroorganismen.

Im folgenden wird die Erfindung detailliert beschrieben, insbesondere in ihren bevorzugten Ausführungsformen. Ferner wird die Erfindung in den Patentansprüchen definiert.

Mit Hilfe von Southern und Northern Blots kann getestet werden, ob bestimmte Bereiche der M. clara Plasmide transkribiert werden. Dazu wird Gesamt-RNA aus Methylomonas clara mit nativen Plasmiden des Mikroorganismus hybridisiert und analysiert.

Zur Gewinnung der erfindungsgemäßen Promotorregion geht man vorzugsweise von dem in der Europäischen Patentanmeldung 98967 beschriebenen Plasmid pBE3 aus Methylomonas clara DSM 2397 aus. pBE3 wird mit Restriktionsenzymen, vorzugsweise EcoRI, AvaI und HincII geschnitten. Anschließend werden die einzelnen Fragmente geblottet und mit radioaktiv markierter Gesamt-RNA hybridisiert, wobei sich herausstellt, daß EcoRI Fragment 3, AvaI Fragment 2 und HincII Fragment 1 starke Transkriptionsaktivitäten besitzen. Andere Fragmente der pBE3-DNA zeigten ebenfalls Wechselwirkung mit einer RNA-Probe aus DSM 2397, jedoch sehr viel schwächer als die genannten Fragmente.

Mit der gleichen Methode kann ebenfalls eine starke Hybridisierung dieser Fragmente mit Gesamt-RNA eines E. coli Stamms, der das Plasmid pBE3 in pBR322 kloniert enthält, nachgewiesen werden.

Mit Promotor-Such-Plasmiden, die für eine leicht nachweisbare Eigenschaft kodieren und insbesondere für E. coli konstruiert sind, können Promotorbereiche auf pBE3 bzw. auf den genannten Fragmenten von pBE3 nach entsprechender Verdauung, bevorzugt beispielsweise mit BalI oder MboI, nachgewiesen werden. Vorzugsweise wird pKK 232-8 als Suchplasmid verwendet, mit dem promotorlosen Strukturgen für das Enzym Chloramphenicol-Acetyltransferase (CAT) zur Inaktivierung von Chloramphenicol. Nach Ligation von pBE3 bzw. dessen Fragmenten in pKK 232-8 und Transformation eines kompetenten E. coli-Stamms, vorzugsweise E. coli HB 101, können auf chloramphenicolhaltigem Nährboden die gewünschten Klone identifiziert werden. Die Plasmid-DNA der chloramphenicolresistenten Klone kann durch Verdauung mit verschiedenen Restriktionsenzymen, beispielsweise MboI, im Falle einer Klonierung von MboI-Fragmenten, oder einer Kombination von EcoRI und BamHI, im Falle einer Klonierung von BalI-Fragmenten, charakterisiert werden. Mit Hilfe von EcoRI und BamHI können aus pBE3-haltigen Hybridplasmiden 4 Fragmente und mit MboI 3 Fragmente identifiziert werden, die jeweils zu einer Expression des zuvor stillen CAT-Gens führen.

Wenn das EcoRI Fragment 3 mit BalI oder MboI geschnitten wird, so kann man mit Hilfe des CAT-Plasmids pKK 232-8 ein Fragment mit ca. 430 bp identifizieren, das mit einem der bei "shot-gun-Klonierung" der gesamten pBE 3 DNA gefundenen Fragmente identisch ist. Dieses Fragment wurde sequenziert, wie auch ein ca. 340 bp großes DNA-Fragment, das nach "shot-gun-Klonierung" der MboI-Fragmente gefunden wurde. Mit Hilfe von kommerziell erhältlichen Computerprogrammen lassen sich die Promotorregionen eingrenzen. Es handelt sich in beiden Fällen um überlappende Promotoren, das heißt, daß ein nach rechts weisender Promotor ($P_R$) sich in seiner Sequenz mit einem nach links weisenden Promotor ($P_L$) überlagert. Die Sequenzen sind in Tabelle 1 dargestellt.

Die sogenannten Consensus-Sequenzen der Promotoren sind nicht wesentlich von den für E. coli Promotoren bekannten Consensus-Sequenzen verschieden und lauten beispielsweise:

a) für die -35-Region:

TTGGTT oder

TTGCGT oder

TTGGCT oder

TTGTTC

b) für die -10-Region:

TATGTT oder

TAAAAT oder

TATGAT.

Zwischen -35-Region und der -10-Region liegt eine beliebige Anzahl von Basen bevorzugt 15 bis 19, die im wesentlichen aus den Basen G und T bestehen. Zwischen der -10-Region und dem Start der Transkription befinden sich ebenfalls eine beliebige Anzahl, bevorzugt ca. 7 Basenpaare. Eine Shine Dalgarno Sequenz ist jeweils vorhanden.

Diese Promotorsequenzen führen sowohl in E. coli als auch in methylotrophen Mikroorganismen, bevorzugt in Methylomonas clara, insbesondere DSM 2397, zur Transkription nachgeschalteter Gene und können in E. coli/M. clara Pendelvektoren zur Expression von Fremdgenen kloniert werden. Besonders bevorzugt werden die in Tabelle I dargestellten Promotorsequenzen des Ball bzw. Mbol-Fragments für derartige Zwecke verwendet.

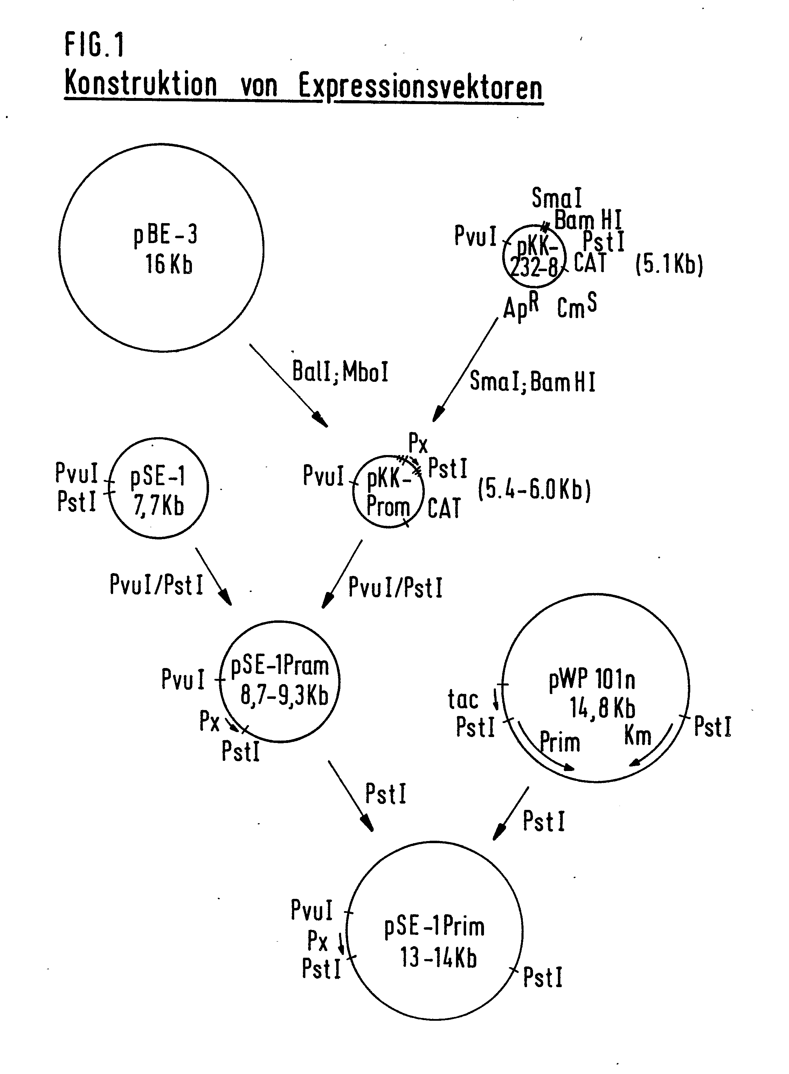

In den folgenden Beispielen wird die Erfindung weitergehend erläutert. Prozentangaben beziehen sich wie auch im Vorangegangenen auf das Gewicht wenn nicht anders angegeben. Die Konstruktion von Expressionsvektoren ist in Figur 1 dargestellt.

**Beispiel 1**

Isolierung von Gesamt-RNA

Bei der Isolierung von intakter Gesamt-RNA wurde in Anlehnung an eine Vorschrift von Zuber und Losick [Cell 35, 275 (1983)] vorgegangen.

Wie bei Gewinnung chromosomaler DNA wird zunächst 1 l Suspensionskultur bei einer $OD_{600}$ von 0,6-0,8 bzw. von 0,8-1,0 bei 4°C zentrifugiert und der Bakterienniederschlag mit 100 ml Puffer (0,15M NaCl; 0,1M EDTA, pH 8,0) gründlich gewaschen, da es ansonsten aufgrund des im Kulturmedium enthaltenen Mohr'-schen Salzes $(NH_4)_2Fe(SO_4)_2$ bei der folgenden Aufarbeitung zur Bildung des tiefroten, eventuell störenden $Fe(SCN)_3$-Komplexes kommt. Sämtliche Isolierungsschritte müssen bei 4°C ausgeführt werden, um den Abbau der sehr labilen mRNA durch zelleigene oder fremde Ribonucleasen zu unterdrücken. Aus diesem Grund erfolgt auch die Resuspension des Bakterienpellets in 40 ml einer Lösung von Guanidiniumthiocyanat und ß-Mercaptoethanol, welche ein starker Inhibitor von Ribonucleasen ist und wie folgt hergestellt wird (Maniatis, et al. Molecular Cloning, A Laboratory Manual, CSH, 1982): 100 g Guanidiniumthiocyanat werden mit 100 ml $H_2O$, 10,6 ml 1M Tris-Cl (pH 7,6) und 10,6 ml 0,2M EDTA (pH 8,0) versetzt und durch Erhitzen auf 70°C unter ständigem Rühren in Lösung gebracht. Nach Abkühlen auf Raumtemperatur und Zusatz von 21,2 ml Sarkosyllösung sowie 2,1 ml ß-Mercaptoethanol wird das Gesamtvolumen mit Wasser auf 212 ml gebracht. Die so hergestellte Lösung kann bei 4°C in einer braunen Glasflasche aufbewahrt werden.

Da Lysozym in einer derart stark denaturierenden und reduzierenden Lösung nicht mehr arbeitet, müssen die resuspendierten Bakterien in der French-Press aufgeschlossen werden. Um das Zerplatzen möglichst aller Zellen zu erreichen, wird die French-Press zwei- bis dreimal hintereinander beschickt und bei je 1400 Psi gefahren (Stempel und Stahlgefäß werden zuvor auf 4°C gekühlt). Das vollständig homogene Lysat wird sofort zweimal phenolisiert, die wäßrige Phase mit einem gleichen Volumen einer 4M wäßrigen LiCl-Lösung gemischt und bei 4°C 1 h stehengelassen. Die hierbei selektiv gefällte und als weißer Niederschlag sichtbare RNA wird bei 3000 rpm (Tischzentrifuge) für 40 min zentrifugiert und in 10 mM EDTA (pH 7,0) aufgenommen. Nach mehrmaligem Phenolisieren wird die RNA in der üblichen Weise mit Ethanol (-20°C)

EP 0 296 484 A2

gefällt und bei -20° C in 70 % Ethanolsuspension aufbewahrt.

Alle verwendeten wäßrigen Lösungen waren 48 h mit 0,1 % Diethylpyrocarbonat-Lösung inkubiert worden, und wurden anschließend mindestens 60 min autoklaviert. Soweit möglich fanden nur sterile Plastikwaren Verwendung (Falcon-Röhrchen Plasti-Pipetten etc.), da auch autokiavierte Glaswaren noch funktionsfähige Ribonucleasen enthalten können.

**Beispiel 2**

Auftrennung von RNA

Zur Elektrophorese von RNA eignen sich sowohl Formaldehydals auch Glyoxalgele mit einer Agarosekonzentration von 0,8 % - 1,2 %. Bei Verwendung eines Glyoxalgels, welches im Gegensatz zu Formaldehydgelen nicht denaturierend ist, wird die RNA durch vorherige Behandlung mit Glyoxal denaturiert. Durch Umpumpen des Puffers wird das durch die Elektrophorese entstehende pH-Gefälle ausgeglichen.

- Formaldehydgele

Zur Herstellung von Agarose-Formaldehydgelen wird eine entsprechende Menge Agarose in Wasser eingewogen und durch Aufkochen im Mikrowellenherd gelöst. Nach Abkühlen auf 60° C wird 1/10 des Endvolumens an 10x Gelpuffer (100 mM MOPS, pH 7,0; 50 mM NaAc; 10 mM EDTA, pH 8,0) und so viel 37% Formaldehydlösung zugefügt, daß sich eine 2,2 M Endkonzentration ergibt [Lehrach et al., Biochemistry $\underline{16}$, 4273 (1977); Goldberg, Proc. Natl. Acad. Sci. $\underline{77}$, 6794 (1980)]. Das flüssige Gel reizt stark die Schleimhäute und muß daher unter dem Abzug gegossen werden. Die in Wasser gelösten oder lyophilisierten RNA-Proben werden mit 20 $\mu$l "heating" puffer (lx Gelpuffer; 50 % Formamid; 2,2 M Formaldehyd) versetzt und 25 min bei 60° C im Wasserbad erhitzt. Nach Mischen mit Auftragspuffer (50 % Glycerin; 1 mM EDTA, pH 8,0; 0,4 % Bromphenolblau) können die Proben auf ein Horizontalgel aufgetragen und bei 80 Volt unter Umpumpen des Laufpuffers elektrophoretisch aufgetrennt werden.

- Glyoxalgele

Bei Verwendung von Glyoxalgelen werden die RNA-Proben durch 60 minütiges Erhitzen bei 50° C in folgendem Mix denaturiert [McMaster und Carmichael, Proc. Natl. Acad. Sci. $\underline{74}$, 4835 (1977)]:

6 M Glyoxal        2,7 $\mu$l
DMSO        8.0 $\mu$l
0,1 M $NaH_2PO_4$ (pH 7,0)        1,6 $\mu$l
RNA (bis zu 20 $\mu$g)        3,7$\mu$l

Die entsprechende Menge Agarose wird durch Erhitzen in 10 mM $NaH_2PO_4$-Lösung (pH 7,0) gelöst, als Gellaufpuffer dient ebenfalls 10 mM $NaH_2PO_4$-Lösung. Die Elektrophorese findet bei 80V und ständigem Umpumpen des Laufpuffers statt.
Sollen RNA-Gele angefärbt werden, so geschieht das erst nach beendeter Elektrophorese. Die Gele werden hierzu entweder in EtBr-Lösung (3$\mu$g/ml) oder in Acridinorangelösung (30$\mu$g/ml) etwa 15 min geschwenkt.

**Beispiel 3**

Spaltung von Plasmid-DNA mit Restriktionsendonucleasen

Einzelverdauungen mit den entsprechenden Enzymen werden in einem Gesamtvolumen von 10-30 $\mu$l bei 37° C durchgeführt. Die Inkubationsdauer beträgt bei einer Enzymkonzentration von 1-3U/$\mu$g DNA in der Regel für gereinigte Plasmid-DNA 1-3 h. Als Puffer finden die von Maniatis (1982) empfohlenen Hoch-, Mittel- und Niedrigsalzpuffer Verwendung. Für Verdauungen mit Sal I wird der vom Hersteller empfohlene

4

Puffer verwendet.

Bei Doppelverdauungen werden die entsprechenden Enzyme meist gleichzeitig zur DNA zugesetzt. Die Vollständigkeit der Verdauungen wird durch Gelelektrophorese eines Aliquots des Reaktionsgemisches auf einem Agarose-Minigel kontrolliert.

Soll Plasmid-DNA aus Schnellaufschlüssen gespalten werden, wird das Reaktionsvolumen auf 40-50 µl gesteigert, um störende Faktoren zu verdünnen. Die Inkubationsdauer beträgt hierbei maximal 1,5 h.

**Beispiel 4**

In vitro $^{32}$P-Markierung von DNA

In einem als Nick-Translation bezeichneten Verfahren kann $^{32}$P-Markierung von doppelsträngiger DNA durch Zugabe von Desoxyribonuclease I, DNA-Polymerase I und radioaktivem $\alpha$-$^{32}$P-dCTP bei gleichzeitiger Anwesenheit der übrigen nichtradioaktiven Nucleotide erreicht werden. [P.W. Rigby et al., J. Mol. Biol. 113, 237 (1977)]:

Zur Markierung von Plasmid-DNA werden üblicherweise 30-50 Ci $\alpha$-$^{32}$P-dCTP eingesetzt, welches entweder in einem Ethanol/Wasser-Gemisch oder in 10fach konzentrierter Form als wäßrige Lösung vorliegt.

Während ethanolhaltiges $\alpha$-$^{32}$P-dCTP bei Raumtemperatur vollständig lyophilisiert wird, kann die wäßrige Form direkt eingesetzt werden. Zum lyophilisierten bzw. zu 5 µl wäßrigem $\alpha$-$^{32}$P-dCTP wird in einem Volumen von 50 µl (bzw. 45 µl) folgendes Gemisch auf Eis zupippettiert:

5 µl 10x "Nick-Translation"-Mix (700 m Tris-Cl;
100 mM MgCl$_2$ 10 mM DTT; 200 µM dTTP;
200 µM dGTP, 200 µM dATP pH 7,4)
200 pg DNase I in wäßriger Lösung
ca. 10 U DNA-Polymerase I aus E. coli.

Nach kurzem Vermischen wird bei 16°C für 60 min inkubiert. Anschließend wird die Reaktion auf Eis durch Zugabe von 5 µl 200 mM EDTA (pH 8,0) abgestoppt. Soglich danach werden auf einer Sephadex-G-50-fine Säule die nichteingebauten Nucleotide von der - nun großenteils markierten - Plasmid-DNA abgetrennt. Hierzu wird die untere Öffnung einer sterilen Pasteurpipette locker mit autoklaviertem Haushaltstuch gestopft und die Pipette mit Sephadex-G-50-fine gefüllt, welches mit TE-Puffer (10 mM Tris, 1 mM EDTA, pH 8,0) äquilibriert ist. Sodann spült man die Säule mit 5 ml TE und trägt das radioaktive Reaktionsgemisch auf. Durch Elution mit TE werden Fraktionen zu je 100 µl in sterilen Eppendorfgefäßen aufgefangen. Mit Hilfe eines Handmonitors können diejenigen Fraktionen ermittelt werden, welche di markierte DNA enthalten. Bei gleichem Säulenvolumen und gleicher Plasmidgröße waren dies in reproduzierbarer Weise die Fraktionen 7 bis 9. Nach Vereinigung dieser Fraktionen wird daraus 1 µl mit 15 ml Szintillationsflüssigkeit (Quickzint, Fa. Zinser) vermischt und im Szintillationszähler die Höhe des radioaktiven Einbaus bestimmt, der in der Regel 2-5x 10$^7$cpm/µg DNA beträgt. Zur Kontrolle der Abtrennung der markierten Plasmid-DNA von nichteingebautem $\alpha$-$^{32}$P-dCTP kann vor der Szintillationsmessung eine TCA-Fällung durchgeführt werden, wobei nur die Plasmid-DNA, nicht aber Nucleotide gefällt werden. Wird neben einem gefällten ein nicht gefälltes Aliquot gezählt, kann aus der Differenz an cpm die Qualität der Trennung erkannt werden.

**Beispiel 5**

Northern Transfer von RNA

Die Übertragung von RNA auf Nitrozellulosefilter erfolgt im wesentlichen wie in Beispiel 10 für DNA beschrieben. Formaldehydgele wie auch Glyoxalgele werden ohne weitere Vorbehandlung direkt mit 20x SSC-Puffer (3 M NaCl; 300 mM Natriumcitrat) geblottet. Die Dauer des Transfers beträgt 36-48 h. Da die Effizienz der Übertragung von RNA wesentlich geringer ist, wenn das Gel mit EtBr oder Acridinorange behandelt wurde, werden zum Northern-Blot nur ungefärbte Agarose-Gele verwendet. Sollen DNA-Marker mithybridisiert werden, werden nach Elektrophorese die entsprechenden Spuren, die die Marker enthalten, ausgeschnitten und - wie beim Southern Transfer beschrieben, - denaturiert, neutralisiert und anschließend wieder in das Gel eingefügt und mitgeblottet.

**Beispiel 6**

Hybridisierung von DNA und RNA

Kurz vor Gebrauch wird der Hybridisierungslösung bis zu einer Endkonzentration von 400 μg/ml Hefe-tRNA oder Kalbsthymus-DNA zugesetzt, welche zuvor durch Erhitzen im siedenden Wasserbad und durch anschließendes Abkühlen in Eiswasser denaturiert wird. Maximal fünf der bei 80° C gebackenen und mit 6x SSC (1 x SSC = 0,15 M NaCl; 0015 M Na-Citrat) befeuchteten Nitrozellulosefilter werden in eine Polyethylen-Klarsichtfolie mit Hybridisierungslösung (ca. 0,1-0 2 ml pro cm$^2$ Filterfläche) unter Vermeidung von Luftblasen eingeschweißt. Um alle unspezifischen, d.h. nicht von DNA-Proben bedeckten Bindungsstellen des Filters abzusättigen, wird für mindestens 12 h im Schüttelwasserbad bei 42° C inkubiert (Prehybridisierung). Danach wird die gesamte Flüssigkeit vollständig entfernt und die Hybridisierungslösung (ca. 30-50 μl pro cm$^2$ Filterfläche) mit der radioaktiven Probe eingefüllt. Diese wird zuvor durch Aufkochen im Hybridisierungsmix und Abkühlen in Eiswasser denaturiert. Die erneut luftblasenfrei verschweißten Filter werden für mindestens 12 h, jedoch nicht länger als 24 h, wie oben bei 42° C inkubiert. Anschließendes Waschen mit je 2x SSC/0,1% SDS für jewells zweimal 15 min befreit die Filter von unspezifischer Radioaktivität. Der die radioaktive Lösung enthaltende Hybridisierungsmix kann bei 4° C aufbewahrt und mehrmals verwendet werden. Nach Trocknen bei Raumtemperatur auf Whatman 3MM-Papier kann mit den Filtern Autoradiographie durchgeführt werden.

**Beispiel 7**

Autoradiographie

Hybridisierte Filter werden auf Whatman 3mm-Papier mit kleinen Klebestreifen fixiert und mit Küchenfolie abgedeckt. Unter Verwendung geeigneter Röntgenfilme (Kodak X-Omat AR; Fuji RX) wird in einer Kassette mit Verstärkerfolie bei -80° C exponiert. Um schärfere Banden zu erzielen kann bei genügend radioaktiv markierten und stark hybridisierenden Proben auch bei Raumtemperatur exponiert werden.

**Beispiel 8**

Auftrennung von DNA

Horizontale Agarose-Gele der Dicke 4-12 mm finden zur Identifizierung von DNA und Auftrennung von DNA- Restriktionsfragmenten Verwendung. Je nach Größe der zu untersuchenden DNA werden Agarosekonzentrationen zwischen 0,4 % - 1,2 % gewählt. Nach Einwiegen der entsprechenden Menge an Agarose in Acetat-Puffer wird im Mikrowellenherd kurz aufgekocht und nach Abkühlen auf 60° C mit EtBr-Lösung (10 mg/ml in Wasser) bis zu einer Endkonzentration von ca. 0,5 μg/ml versetzt. Übliche Elektrophoresebedingungen sind 20-50V bei einem Stromfluß von 15-30 mA. Enthalten die Gele selbst kein EtBr, erfolgt die Anfärbung nach beendeter Elektrophorese in einer Ethidiumbromidlösung der Konzentration 3 μg/ml. DNA-Banden können durch Bestrahlen mit UV-Licht der Wellenlänge 254nm sichtbar gemacht und auf Polaroid-Film (Typ 107) fotographiert werden.

**Beispiel 9**

Southern Transfer von DNA

Die Übertragung von DNA aus Agarose-Gelen wird nach Southern [J. Mol. Biol. 98, 503 (1975)] durchgeführt: Das Agarose-Gel wird zunächst 1 h in Denaturierungspuffer (1,5M NaCl; 0,5M NaOH), dann 2 h im Neutralisierungspuffer (0,5M Tris-Cl, pH 5,5; 0,9M NaCl) geschwenkt. Der pH-Wert der Neutralisierungslösung bzw. des Gels darf danach nicht über 7,5 liegen.
Mehrere Whatman 3MM Papierstreifen werden so über einen Plastikrost gezogen, daß sie durch Eintauchen in ein Vorratsgefäß mit 20x SSC die Flüssigkeit hochsaugen. Auf diesen Rost wird ein mit 2x SSC angefeuchtetes Whatman 3MM Papier in der Größe des Agarosegels gelegt und darauf das Gel selbst, jedoch in umgekehrter Position, so daß die ursprüngliche Unterseite nach oben zu liegen kommt. Auf das Gel werden nun ein gleichgroßer in 6x SSC getauchter Nitrozellulosefilter und darüber zwei ebenfalls gleichgroße Whatman 3MM Papierbögen gelegt. Vorhandene Luftblasen zwischen den einzelnen Schichten müssen durch vorsichtiges Andrücken sorgfältig entfernt werden. Obenauf wird ein Stapel Papierhandtücher gelegt, um den 20x SSC-Puffer durch das Agarose-Gel nach oben zu saugen, wodurch der Transfer der DNA auf den Nitrozellulosefilter bewirkt wird. Die Ränder des Filters können mit ca. 3 cm breiten Plastikstreifen abgedeckt werden, um einen Kurzschluß zwischen dem SSC-Reservoir und den Papierhandtüchern zu vermeiden. Bis ein großer Teil der Feuchtigkeit aus dem Gel nach oben diffundiert ist, wird der Aufbau nur mit einer Glasplatte beschwert. Nach ca. 1 h kann dann ein Gewicht von etwa 1 kg aufgelegt werden.
Je nach Größe und Menge der zu blottenden DNA ist der Transfer nach 15-24 h beendet. Das nun stark geschrumpfte Gel wird samt Filter umgedreht, so daß mit einem weichen Filzstift durch die Auftragskammern die DNA-Auftragsstellen markiert werden können. Anschließend wird der Filter 5 min in 6x SSC geschwenkt und auf einem Whatman 3MM Papier bei Raumtemperatur getrocknet und 2-4 h bei 80°C gebacken, um die DNA zu fixieren.

**Beispiel 10**

Phosphatase-Reaktion

Der linearisierte, in TE (10 mM Tris-Cl, 1 mM EDTA, pH 8,0) gelöste Vektor wird mit 12,5 $\mu$l 20x RAP-Puffer (800 mM Tris-Cl, 100 mM MgCl$_2$, 2 mM ZnCl$_2$, pH 8,0) und Wasser auf in Endvolumen von 250 $\mu$l gebracht und mit 5OU CIP (Calf Intestinal Phosphatase) versetzt. Nach Inkubation bei 37°C für 3 h wird nach Zugabe von 5 $\mu$l 10 % SDS für 15 min auf 68°C erhitzt. Nach dreimaligem Phenolisieren erfolgt Ethanolfällung und erneute Aufnahme in TE.

**Beispiel 11**

5′-Markierung von RNA mit radioaktivem Phosphat

Zur radioaktiven Markierung von RNA wurden etwa 5 $\mu$g der nach Beispiel 1 isolierten Gesamt-RNA lyophilisiert und in 10 $\mu$l Puffer (50mM Tris-Cl, 5mM Glycin, 10 Spermidin x 3HCl, 10mM EDTA, pH 9,5) resuspendiert. Durch zehnminutiges Erhitzen auf 90°C wurde die RNA in Bruchstücke mit freien 5′-Enden gespalten und anschließend auf Eis gestellt. Dann wurden folgende Lösungen zugegeben:
10 $\mu$l 5x Kinase-Puffer (125 mM Tris-Cl, 50 mM MgCl$_2$, 10 mM DTT, pH9,5)
17 $\mu$l H$_2$O
3 $\mu$l T4-Polynukleotid-Kinase (Boehringer Mannheim bzw. Biolabs)

10 $\mu$l $^{32}$P $\gamma$ ATP (10 $\mu$Ci/$\mu$l, Amersham)

Nach Inkubation für eine Stunde bei 37°C wurde die radioaktiv markierte RNA durch Chromatographie uber Sephadex G 50 vom $\gamma$-$^{32}$P-ATP abgetrennt. Durchschnittlich wurde eine RNA mit $10^7$cpm/$\mu$g erhalten. Zur Hydridisierung wurde alles eingesetzt.

**Beispiel 12**

Klonierung von Promotorfragmenten aus M. clara

Zur Klonierung von Promotorfragmenten aus M. clara wurde das Promotor-Suchplasmid pKK232-8 (Brosius, Gene, 27 (1984) 151-160), mit den Restriktionsenzymen Smal bzw. BamHI wie unter Beispiel 3 beschrieben vollständig verdaut. Im Falle des BamHI verdauten pKK232-8 wurde noch eine Behandlung mit alkalischer Phosphatase angeschlossen. In die entsprechend vorbehandelten Vektoren wurden Restriktionsfragmente ligiert, die durch Verdauung des M. clara Plasmids pBE-3 erhalten worden waren. Die Restrikionsverdauung dieser Plasmide wurde mit den Enzymen Ball sowie Mbol jeweils einzeln und vollständig durchgeführt. Nach Ligation der jeweiligen Mischungen wie unter Beispiel 13 beschrieben wurden 10 $\mu$l Aliquots aus den Ligase-Ansätzen entnommen und kompetente Zellen des E. coli Stammes HB101 nach der Methode von Cohen et al. [Proc. Natl. Acad. Sci. 69, 2110 (1972)] mit diesen Aliquots transformiert. Selektioniert wurde auf L-Broth Platten, die 100 $\mu$g/ml Chloramphenicol enthielten. (In dem Vektor pKK232-8 ist ein intaktes Chloramphenicol Resistenzgen enthalten, das jedoch wegen eines fehlenden Promotors nicht abgelesen wird). Resistente Kolonien wurden gepickt und durch Minilyse auf den Gehalt an Plasmid-DNA überprüft. Kolonien mit Plasmiden, die einen Größenzuwachs zeigten, wurden gepickt und die Plasmide nach Verdauung mit den Restriktionsenzymen EcoRI/BamHI (im Falle der Klonierung in die Smal Schnittstelle von pKK232-8) bzw. Mbol allein (im Falle der Klonierung in die BamHI Schnittstelle von pKK232-8) durch Elektrophorese in einem 8 %igen PAA-Gel auf die Anwesenheit von neuen Restriktionsfragmenten getestet.

**Beispiel 13**

Ligase-Reaktion

Das Ligieren von isolierten DNA-Fragmenten und Plasmid-Vektor erfolgte unter den von Maniatis (1982) empfohlenen Bedingungen.

Die Reaktion wird im entsprechenden Ligase-Puffer bei einer DNA-Konzentration von 200-400 $\mu$g/ml in einem Volumen von 10-20 $\mu$l ausgeführt.

Zu diesem Ansatz werden 200-400 U T4-Ligase gegeben (dabei entspricht 1 U der Menge Enzym, die nötig ist, um 50 % einer Hind III verdauten DNA in 30 min. bei 16°C in einem Volumen von 20 $\mu$l zu ligieren).

Die Inkubation erfolgt bei 14-16°C mindestens 24 h. Anschließend wird zur Kontrolle dr Ligierung 1/10 Volumen des Ligase-Ansatzes entnommen und auf einem 0,4 % Agarose-Gel mit einer Probe verglichen, die vor Zugabe der T4-Ligase dem Reaktionsgemisch entnommen und bei 4°C aufbewahrt wurde.

**Beispiel 14**

Klonierung von Promotorfragmenten in M. clara/E. coli Pendelvektoren.

Die in das Promotor-Suchplasmid pKK232-8 klonierten Fragmente mit Promotoraktivität können durch eine Doppelverdauung mit den Enzymen Pstl und Pvul auf einem DNA-Fragment ausgeschnitten werden, das neben den interessierenden Promotor-Sequenzen noch einen Anteil aus dem Vektor pKK232-8 enthält.

Aus dem M. clara/E. coli Hybridvektor pSE-1 (gemäß Deutscher Patentanmeldung P 36 35 583) fällt bei PstI/PvuI Doppelverdauung ein kleines Fragment heraus, das durch das aus pKK232-8 stammende, Promotor-tragende DNA-Segment ersetzt werden kann. Die verschiedenen pKK232-8 Abkömmlinge, die unterschiedliche DNA-Segmente aus M. clara mit Promotoraktivität enthalten, werden mit den Enzymen PvuI und PstI einer Doppelverdauung unterzogen. Zusätzlich wird noch mit dem Enzym PvuII verdaut, um in der anschließenden Ligase-Reaktion eine Zurückligierung des Ausgangsplasmids zu vermeiden. Hierzu werden alle drei Enzyme gleichzeitig in einem Puffer (50 mM Tris-Cl, 10 mM NaCl, 10 mM MgCl$_2$, 10 mM DTT, pH 7,6) der DNA zugesetzt und eine Stunde bei 37°C inkubiert. Parallel dazu wird der Vektor pSE-1 mit den Enzymen PvuI und PstI vollständig wie oben beschrieben verdaut. Aliquots der jeweiligen Ansätze werden nach erfolgter Reaktion durch Agarose-Gelelektrophorese überprüft und die Vollständigkeit der Reaktionen festgestellt. Sodann werden Aliquots der Ansätze zusammengegeben und miteinander wie in Beispiel 13 beschrieben ligiert. Nach erfolgter Ligase-Reaktion werden kompetente Zellen des E. coli Stammes HB101 mit dem Ligase-Ansatz transformiert und Tetracyclin-resistente Kolonien selektioniert. Durch Minilyse und Verdauung der isolierten Plasmid-DNAs mit den Enzymen PstI/PvuI werden die Klone selektioniert, die das gewünschte Fragment aus pKK232-8 in den Vektor pSE-1 kloniert enthalten.

**Beispiel 15**

Klonierung des Primasegens in Pendelvektoren mit M. clara Promotoren

Die Abkömmlinge des Plasmids pSE-1 mit Promotoren aus M. clara enthalten eine singuläre PstI Schnittstelle, in deren Richtung die Transkription vom Promotor aus gerichtet ist. Bei Klonierung eines Strukturgens in die einzige PstI Schnittstelle dieser pSE-1 Abkömmlinge kann daher eine Transkription dieses Strukturgens ausgehend vom klonierten Promotor erfolgen. Als Quelle eines promotorlosen Strukturgens dient das Plasmid pWP101n, Fürste et al. [Gene, 48, 119 1986)] aus dem durch PstI Verdauung ein DNA-Fragment gewonnen werden kann, auf dem das Strukturgen für das Enzym Primase ohne Promotor vorhanden ist. Das in pWP101n enthaltene PstI Fragment, auf dem das Primase-Gen enthalten ist, stammt ursprünglich aus dem konjugativen Plasmid R751 und kann wegen einer gleichzeitig vorhandenen Kanamycin-Resistenz einfach selektioniert werden. Dieses PstI Fragment aus pWP101n kann zunächst in die einzige PstI Schnittstelle des E. coli Vektors pBR322 kloniert, wobei wie in den Beispielen 3 und 13 beschrieben vorgegangen wird. Nach erfolgter Ligation werden kompetente E. coli HB101 Zellen transformiert, Tetracyclin resistente Klone selektioniert und durch Überprüfung der Ampicillin-Resistenz Klone mit Einbau aufgefunden und das Vorhandensein des aus pWP101n stammenden PstI Fragments durch Minilyse und Verdauung mit Restriktionsenzymen bestätigt. Die erhaltenen Klone mit dem PstI Fragment aus pWP101n in beiden möglichen Richtungen dienen als Quelle für das das Primasegen tragende PstI-Fragment.
Ausgehend von diesen Plasmiden kann das PstI-Fragment mit promotorlosem Strukturgen der Primase auf pSE-1 Derivate mit verschiedenen Promotoren aus M. clara übertragen werden. Hierzu werden diese pSE-1 Derivate in Analogie zu Beispiel 3 mit dem Restriktionsenzym PstI vollständig verdaut, was zu einer Linearisierung dieser Plasmide führt. Parallel dazu wird das promotorlose Strukturgen der Primase durch vollständige Verdauung mit PstI aus dem E. coli Vektor pBR322 herausgelöst. In den gleichen Ansatz wird zusätzlich noch das Enzym SalI gegeben, um in der nachfolgenden Ligase-Reaktion eine Rezirkularisation des Vektors pBR322 zu verhindern. Aliquots der beiden Ansätze werden zusammengegeben und wie in Beispiel 13 beschrieben miteinander ligiert. Wie in Figur 1 dargestellt, kann man auch so vorgehen daß man die pSE-1 Derivate und pSP101n mit PstI schneidet und direkt miteinander ligiert. Nach erfolgter Inkubation wird der Ligase-Ansatz verwendet, um kompetente E. coli HB101 Zellen zu transformieren. Tetracyclinresistente Kolonien werden selektioniert und die enthaltene Plasmid-DNA nach Minilyse auf das Vorhandensein der gewünschten DNA-Anteile überprüft. Kolonien, die das PstI Fragment mit dem promotorlosen Primase-Gen in pSE-1 kloniert enthalten, können durch PstI Verdauung der jeweiligen Plasmid-DNAs identifiziert werden. Die Orientierung des PstI Fragments mit dem Primasegen relativ zum Vektor ergibt sich durch eine Verdauung mit EcoRI. Auf diese Art können Klone gefunden werden, die das Primase-Gen in einer solchen Orientierung enthalten, daß es durch die vorgeschalteten Promotorsequenzen aus M. clara transkribiert werden kann.

**Beispiel 16**

Subklonierung in pUCl2

Der E. coli Sequenziervektor pUCl2 wird mit den Restriktionsenzymen AccI, SmaI, BamHI bzw. EcoRI wie unter Beispiel 3 beschrieben vollständig verdaut und wie unter Beispiel 10 beschrieben mit alkalischer Phosphatase behandelt. Das zu sequenzierende DNA-Fragment wird mit den Restriktionsenzymen MspI, TaqI, Sau3A, RsaI, HaeIII, BalI bzw. NotI gespalten. Die hierdurch gebildeten kleinen DNA-Fragmente werden mittels Elektrophorese durch 1 %ige Agarose in TAE-Puffer (40 mM Tris-Acetat, 5 mM Na-Acetat, 1 mM EDTA, pH 8,0) aufgetrennt und durch Bindung an Membranfilter (Schleicher und Schüll, NA-45 DEAE wie vom Hersteller beschrieben einzeln isoliert (Schleicher und Schüll, Sequences, Application Update 364). Im Falle der NotI Fragmente wurden die überhängenden Enden durch Inkubation mit DNA-Polymerase-(Klenow) in Gegenwart aller 4 Nukleotide wie bei Maniatis et al. (1982 beschrieben aufgefüllt.

Die isolierten Fragmente, die nach Verdauung mit den jeweiligen Restriktionsendonukleasen entstanden waren, werden in Ligase-Ansätzen dergestalt mit dem gespaltenen Vektor pUCl2 kombiniert, daß die jeweils gebildeten kohäsiven oder stumpfen Enden zueinander passen. Die einzelnen Ansätze werden durch Inkubation mit dem Enzym T4-DNA-Ligase wie unter Beispiel 13 beschrieben ligiert und in kompetente Zellen des Stammes E. coli JM83 transformiert. Ampicillin resistente und nicht blau gefärbte Kolonien wurden auf LB-Agar, der 50 μg/ml Ap und 50 μg/ml X-Gal enthielt, isoliert. Durch Minilyse und anschließende Agarose-Gelelektrophorese wird der Einbau der gewünschten Fragmente überprüft.

**Beispiel 17**

Sequenzierung

Die Sequenzierung der in pUC12 klonierten Fragmente wird nach der von der Firma Boehringen Mannheim herausgegebenen Arbeitsanleitung (Guidelines for quick and simple plasmid sequencing, 1986) durchgeführt.

**Beispiel 18**

S1-Mapping

Die Festlegung des Transkriptionsstarts erfolgt wie in Maniatis et al., S. 207-209 bzw. in Favaloro et al., Methods Enzymology 65 (1980), S. 718ff beschrieben, mit der Abweichung, daß das nach S1 Verdauung gebildete DNA-RNA Hybrid auf einem Sequenzgel analysiert wird.

Tabelle 1

P_R:

-35

5' GATCCTCTAC GGTCGACCTC AAGCCCAGCC ATTGGCTGCA

-10

AGCCGAAGCA TCATATGATC CGGCAGGTGG GAACAACCTT

Mbo I.  GCCACCCCCG TGCAACCTCA ACTCTTTGTG 3'


P_L:

-35

5' CACAAAGAGT TGAGGTTGCA CGGGGGTGGC AAGGTTGTTC

-10

CCACCTGCCG GATCATATGA TGCTTCGGCT TGCAGCCAAT

GGCTGGGCTT GAGGTCGACC GTAGAGGATC 3'


P_R:

-35

GATCGCTTCG CTTGCCATGT CAGACTCCTT CCGGTTGGTT

-10

GTGTTGTTTG TTGTTGTATG TTGTATTTTA CAGTAAACAA

BalI.  CAACAAACGC AACAGGCGAA GGCCGGCCGG ATG


P_L:

5' GGCGCGGCCG CCGCCGACAT CCGGCCGGCG CTTCGCCTGT

-35                        -10

TGCGTTTGTT GTTGTTTACT GTAAAATACA ACATACAACA

ACAAACAACA CAACCAACCG GAAGGAGTCT GACATGGCAA

GCGAAGCGAT 3'

**Ansprüche**

1. Promotorregion, aus einem nativen Plasmid von Methylomonas clara, zur Expression von Genen in E. coli und methylotrophen Mikroorganismen.

2. Promotorregion nach Anspruch 1, gekennzeichnet durch eines oder mehrere der folgenden Merkmale
a) die -35 Region im Promotor hat die Nucleotidsequenz
TTGGTT oder
TTGCGT oder
TTGGCT oder
TTGTTC,
b) die -10 Region hat die Nucleotidsequenz
TATGTT oder
TAAAAT oder
TATGAT,
c) zwischen der -35 Region und der -10 Region ist eine Spacergruppierung von 15 bis 19 Basenpaaren.

3. Promotorregion nach Anspruch 1 oder 2 aus dem Plasmid pBE-3 aus Methylomonas clara DSM 2397.

4. Promotorregion nach einem oder mehreren der Ansprüche 1 bis 3, gekennzeichnet durch eine der in Tabelle 1 aufgeführten Sequenzen.

5. Hybridvektor, enthaltend eine Promotorregion nach einem oder mehreren der Ansprüche 1 bis 4.

6. E. coli oder methylotrophe Mikroorganismen enthaltend einen Hybridvektor nach Anspruch 5.

7. Polypeptid, exprimiert von E. coli oder methylotrophen Mikroorganismen nach Anspruch 6.

Patentansprüche für folgende Vertragsstaaten: ES, GR

1. Verfahren zur Herstellung einer Promotorregion zur Expression von Genen in E. coli und methylotrophen Mikroorganismen, dadurch gekennzeichnet, daß aus nativen Plasmiden von Methylomonas clara eine Promotorregion mit einem oder mehreren der folgenden Merkmale isoliert wird:
a) die -35 Region im Promotor hat die Nucleotidsequenz
TTGGTT oder
TTGCGT oder
TTGGCT oder
TTGTTC,
b) die -10 Region hat die Nucleotidsequenz
TATGTT oder
TAAAAT oder
TATGAT,
c) zwischen der -35 Region und der -10 Region ist eine Spacergruppierung von 15 bis 19 Basenpaaren.

2. Verfahren nach Anspruch 1, dadurch gekennzeichnet, daß die Promotorregion aus dem Plasmid pBE-3 aus Methylomonas clara DSM 2397 isoliert wird.

3. Verfahren nach Anspruch 1 oder 2, dadurch gekennzeichnet, daß die Promotorregion mit den in Tabelle 1 aufgeführten Sequenzen isoliert wird.

4. Verfahren zur Expression von heterologen Genen in E. coli und methylotrophen Mikroorganismen, dadurch gekennzeichnet, daß man in Ableserichtung vor das heterologe Gen eine Promotorregion mit Shine-Darlgarno-Sequenz setzt, deren
-35 Region im Promotor die Nucleotidsequenz
TTGGTT oder
TTGCGT oder
TTGGCT oder
TTGTTC hat,

-10 Region im Promoter die Nucleotidsequenz
TATGTT oder
TAAAAT oder
TATGAT hat, und
zwischen deren -35 Region und der -10 Region eine Spacergruppierung von 15 bis 19 Basenpaaren ist.

5. Verfahren nach Anspruch 4, dadurch gekennzeichnet, daß man anstelle der Shine-Darlgarno-Sequenz der Promotorregion eine dem heterologen Gen angehörige ribosomale Bindungsstelle verwendet.

FIG.1
Konstruktion von Expressionsvektoren